# EUROPEAN PATENT APPLICATION

(11) **EP 4 215 218 A1**
(43) Date of publication of application: **26.07.2023**
(21) Application number: 23020038.8
(22) Date of filing: 23.01.2023
(51) Int. Cl.: A61L 2/26, A61L 11/00, A61L 2/06, A61L 2/07

(54) **WASTE STERILISATION CELL**

(30) Priority: 24.01.2022 IT 202200001103
(71) Applicant: Newster System S.r.l., 47853 Cerasolo di Coriano (RN) (IT)
(72) Inventor: Casalboni, Giorgio, 47921 RIMINI (RN) (IT); Catapano, Enrico, 47923 RIMINI (RN) (IT); Acquaviva, Matteo, 47923 RIMINI (RN) (IT); Copertaro, Benedetta, 62019 RECANATI (MC) (IT); Trevisson, Laura, 47921 RIMINI (RN) (IT); Pericoli, Marco, 47923 RIMINI (RN) (IT); Magrini, Gianluca, 47838 RICCIONE (RN) (IT)
(74) Representative: Montebelli, Marco

(57) **Abstract**

A waste sterilisation cell comprises a hollow main body (30), suitable for containing the waste, and a sealable lid (50): inside the main body (30) there is a rotor (40) provided with blades (41) and on its surface there is a first plurality of seats (60a, 60b, 60c), suitable for containing a plurality of sensors (61a, 61b, 61c), so as to verify that the predetermined temperature is reached inside the cell (20).

## Description

This invention relates to a waste sterilisation cell.

For waste sterilisation or disinfection, in particular of infected hospital waste, use is known of an apparatus which comprises a sealable metal cell, provided with a lid, into which the waste is loaded. Positioned inside the cell there is a rotor on which blades are mounted for grinding the waste. Positioned in the lower part of the cell there are also steel stationary contact elements which operate in conjunction with the blades for striking, breaking up and disintegrating the material, also converting the kinetic energy into thermal energy. The rotor is driven at a speed such that it generates by friction, and maintains, inside the cell a temperature sufficient for sterilisation or disinfection of the waste. The temperature inside the cell is detected and controlled by a sensor which can drive the opening of a solenoid valve and cause water to be injected into the cell.

During the disinfection process a slight negative pressure is maintained in the cell. After the rotor has started operating, when approximately 100°C is reached the temperature remains stable until the water present in the waste has completely evaporated, then the temperature reaches a peak of approximately 150°C, the rotor slows and cooling water is injected, by one or more nozzles located on the wall of the cell, to lower the temperature to approximately 95°C, after which the residue is automatically unloaded.

In the prior art apparatus, the malodorous vapours released during the treatment cycle are cooled and then treated in a filtering device. To cool the vapours, in the prior art apparatus a liquid separator (demister) is used, made up of two exchangers in which the vapour is first condensed by injecting running water and then further cooled.

The prior art apparatus therefore has several disadvantages: in addition to considerable water consumption, the sterilisation cell generates very malodorous vapours; moreover, the presence of only one temperature sensor means that control of the progress of the sterilisation is not very precise and does not provide sufficient guarantees regarding the effectiveness of the procedure, should the sensor malfunction.

At present, the structure of the cutting unit mounted on the rotor inside the cell comprises a blade holder which supports blades positioned horizontally along its lateral edges, that is to say, extending along a line perpendicular to the axis of the rotor, and a pair of blades positioned vertically, that is to say, along a line parallel to the axis of the rotor. The purpose of latter is, above all, to tear the plastic covers in which the waste is usually contained when it is inserted into the cell.

However, it was observed that even a such a structure has several disadvantages: first, on one hand the arrangement of the blades only on the lateral edges of the blade holder limits the cutting surface, reducing the theoretical effectiveness of the blades, and on the other hand, the fact that the head of the blade holder makes contact with the waste to be sterilised at high temperatures causes a further quite rapid deterioration of the blade holder.

Moreover, the vertical blades substantially describe a circle and it may be the case that they are not very effective at tearing the plastic covers which contain the waste.

The aim of this invention is therefore to eliminate the above-mentioned disadvantages.

The invention, characterised as set out in the claims, achieves the aim thanks to various improvements made to the structure of the sterilisation cell.

The main advantage is essentially the fact that the modifications made to the lid of the cell allow a reduction in water consumption and the bad smell produced during waste sterilisation; moreover, the opening for inserting waste into the cell is also increased.

Advantageously, other improvements allow more precise monitoring of the distribution of the temperatures inside the cell during the sterilisation process, supplying greater guarantees regarding the effectiveness of the process. The new shape of the blades allows better fragmentation of the waste and of the plastic covers which contain it, together with longer lasting blade supports.

This invention will now be described with reference to the accompanying drawings, which illustrate an example, non-limiting embodiment of it, in which:
- Figure 1 is a schematic perspective side view of the invention;
- Figure 2 is a side view of the invention;
- Figure 3 is a cross-section of the invention according to III - III from Figure 2.
- Figure 4 is an enlarged detail from Figure 3;
- Figure 5 is a top view of the invention;
- Figure 6 is a perspective top view of a detail of the invention;
- Figure 7 is a perspective bottom view of the detail from Figure 6.

As shown in the figures, the invention relates to a waste sterilisation cell which, unlike autoclaves, operates in a condition with a slight negative pressure.

It comprises a hollow main body 30, suitable for containing the waste, and a sealable lid 50. Inside the main body 30 there is a rotor 40 provided with blades 41, whilst on its lateral surface there is a first plurality of seats 60a, 60b, 60c, suitable for containing a plurality of sensors 61a, 61b, 61c, appropriate for verifying that predetermined temperatures are reached and maintained for predetermined periods of time.

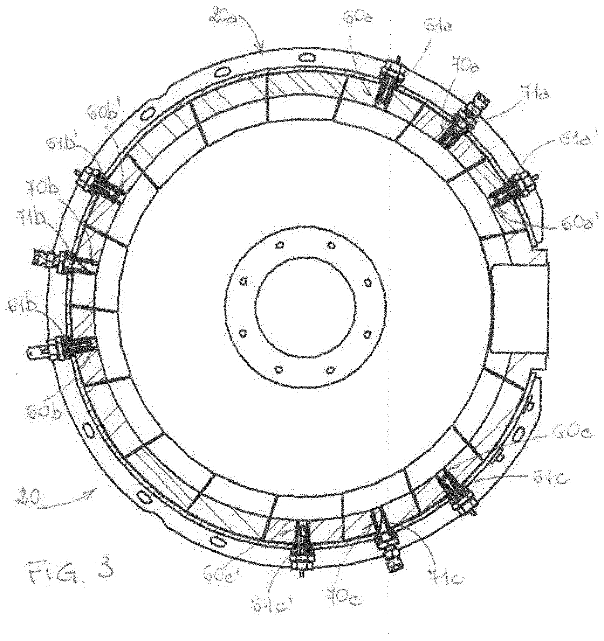

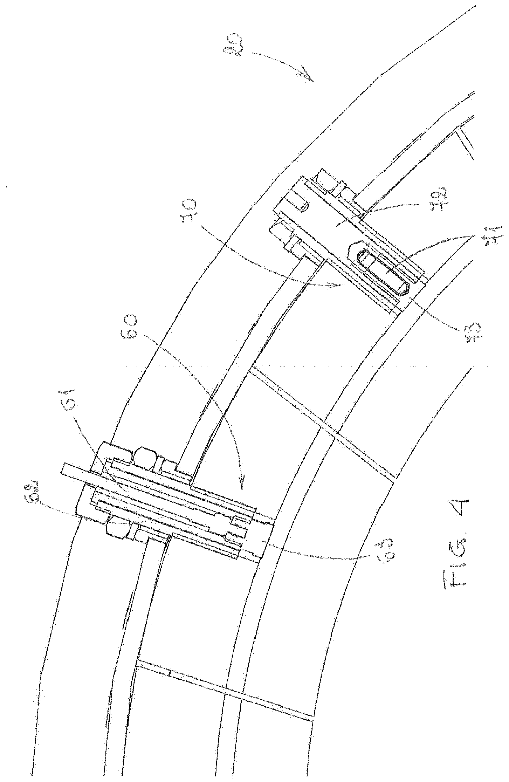

As shown in Figures 3 and 4, in a non-limiting preferred embodiment there are three seats 60a, 60b, 60c which are evenly distributed, that is to say, approximately 120° from each other, along the lateral surface of the main body 30, in such a way that the sensors 61a, 61b, 61c effectively cover the inner surface of the cell 20.

In the example shown, as can be seen in the enlarged view in Figure 4, the seats 60a, 60b, 60c comprise an insulating cover 62, which wraps around a corresponding sensor 61, suitable for avoiding contact between the sensor 61 and the walls 20a of the cell 20, and a metal cap 63, leading towards the inside of the cell 20, suitable for being in contact both with the waste and with the sensor 61: in this way, direct contact between the sensors 61 and the waste is avoided, preserving their integrity, but in any case it is guaranteed that the temperature measured is not greater than that of the waste itself.

Figure 3 also shows an additional set of seats 60a', 60b', 60c' and of relative sensors 61a', 61b', 61c' for allowing confirmation measurements of the temperatures values detected inside the cell 20.

Figures 3 and 4 also show a second plurality of seats 70a, 70b, 70c, suitable for containing a plurality of biological indicators 71a, 71b, 71c, in such a way as to test the sterilisation which took place.

In this case too, as can be seen in Figure 4, the seats 70a, 70b, 70c of the biological indicators 71a, 71b, 71c comprise an insulating cover 72, which wraps around a corresponding biological indicator 71, suitable for avoiding contact between the biological indicators 71a, 71b, 7c and the walls 20a of the cell 20, and a metal cap 73 leading towards the inside of the cell 20, suitable for being in contact both with the waste and with the biological indicator 71.

This allows verification of the capability of the cell 20 for sterilising potentially infectious medical waste: in fact, by subjecting the biological indicators 71 containing heat-resistant bacterial spores to a sterilisation cycle then comparing them with other control indicators whose microbial inactivation is known, it will be possible to establish whether or not the sterilisation was successful.

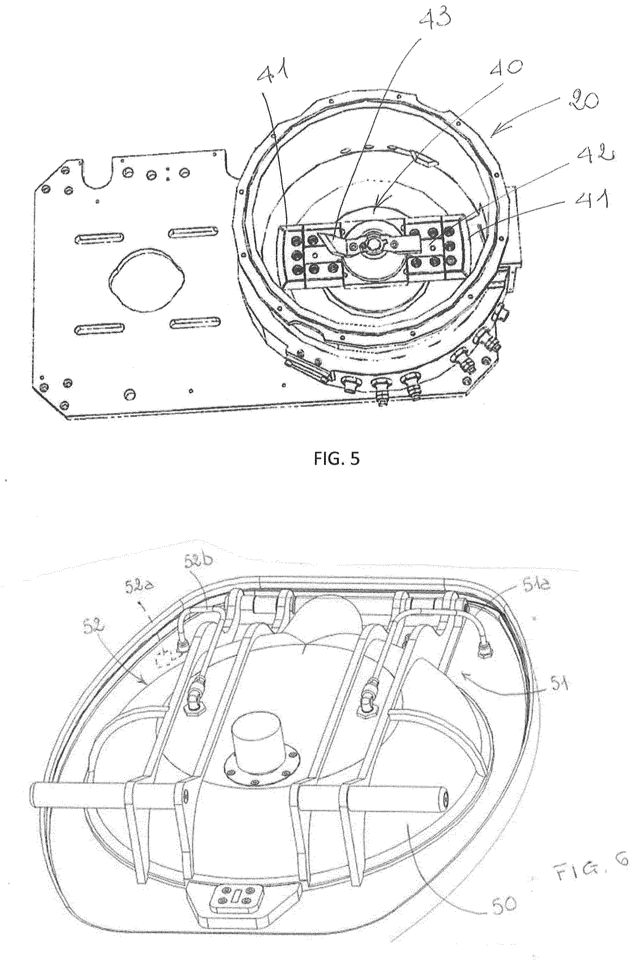

Figure 5 shows how, in the lower central part of the cell 20, the rotor 40 supports at least one blade holder 42 provided with at least one blade 41 oriented perpendicularly to the axis of the rotor 40: the blade 41, which may be made in a single body or in multiple juxtaposed segments, is distributed on the entire outer edge of the blade holder 42, so as to protect it from contact with the waste and to limit wear on it thereby making it longer lasting. In the example shown a single blade holder 42 is used which supports a pair of aligned blades 41, but other configurations could also be used with a different number of blades 41 and blade holders 42.

The rotor 40 also supports at least one blade 43 oriented upwards, preferably angled at an angle of between 30° and 70° relative to the blade holder 42, which is particularly useful for tearing the plastic covers in which the waste is usually contained when it is inserted into the cell 20.

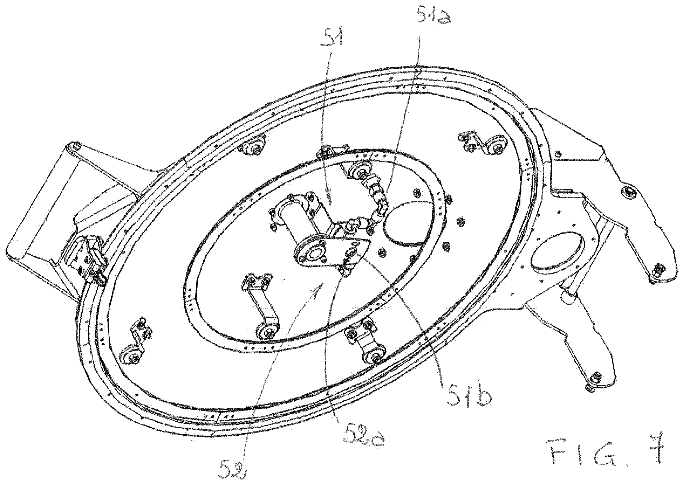

Finally, the lid 50 of the cell 20, shown in Figures 6 and 7, comprises an air cooling device 51, suitable for inserting air into the cell 20 and an enzymatic deodorising device 52, suitable for controlling the smell of the waste and of the vapours.

The air cooling device 51 comprises a duct 5 1a for inserting air and a nebuliser 5 1b from which the air is inserted into the cell 20, the enzymatic deodorising device 52 comprises a liquid deodorant tank 52a and a duct 52b leading towards the nebuliser 51b.

When the sterilisation temperature, of approximately 150°C, has been reached system water is injected into the sterilisation cell 20 by means of a plurality of nozzles 20b, connected to the water system, which are located on the walls 20a of the cell 20, in order to avoid a temperature which is too high from being reached: upon making contact with the waste, which holds at a temperature of approximately 150 °C, the water immediately passes to the vapour state creating an environment saturated with vapour which guarantees complete sterilisation of the waste itself. When the rotor 40 starts to turn more slowly, and the cooling step starts, water is inserted by the nozzles 20b which are located on the walls 20a of the cell 20; then the insertion of water by the nozzles 20b stops until the temperature reaches 145°C. At this point activation of injection of liquid deodorant from the tank 52a occurs which, mixed with the cold air, is nebulised and inserted into the cell 20, maintaining a moist environment until the end of cycle temperature of approximately 95°C is reached.

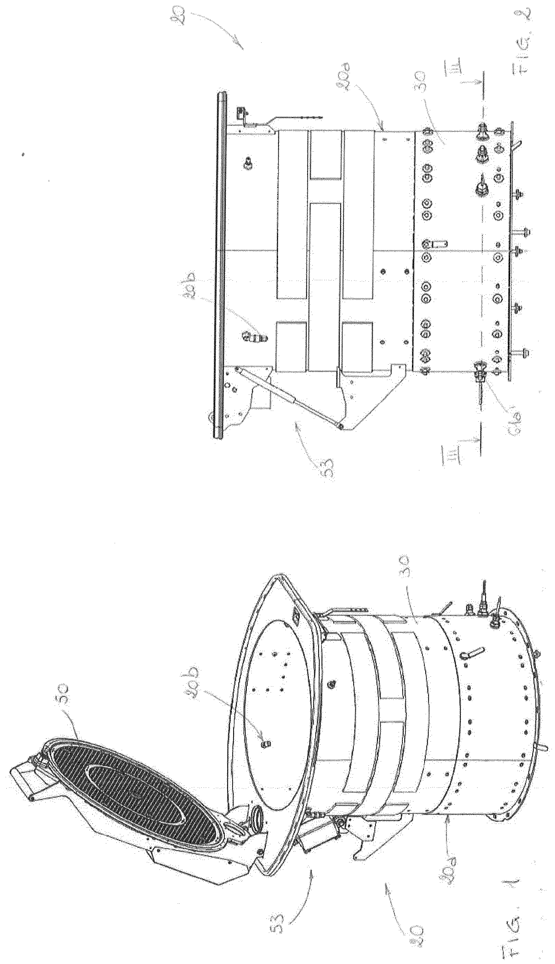

This mechanism guarantees that the waste remains in an environment saturated with vapour with a temperature higher than 135°C for a predetermined sterilisation time. As can be seen in Figure 1, the cell 20 also comprises a device 53 for automatically opening the lid 50, so as to facilitate, and if necessary to automate, insertion of the waste.

## Claims

1. A waste sterilisation cell, comprising a hollow main body (30), suitable for containing the waste, inside which there is a rotor (40) provided with blades (41), and a sealable lid (50), **characterised in that** the main body (30) comprises a plurality of sensors (61a, 61b, 61c), housed in a corresponding first plurality of seats (60a, 60b, 60c), so as to monitor the distribution of the temperature inside the cell (20).

2. The cell according to claim 1, **characterised in that** the main body (30) comprises a plurality of biological indicators (71a, 71b, 71c), housed in a corresponding second plurality of seats (70a, 70b, 70c), in such a way as to test the sterilisation which took place.

3. The cell according to claim 1, **characterised in that** the rotor (40) supports at least one blade holder (42) provided with at least one blade (41) oriented perpendicularly to the axis of the rotor (40), distributed on the entire outer edge of the blade holder (42), so as to protect it from contact with the waste.

4. The cell according to claim 2 or 3, **characterised in that** the rotor (40) supports at least one blade (43) oriented upwards.

5. The cell according to claim 1 or 2 or 3, **characterised in that** the lid (50) comprises an air cooling device (51), suitable for inserting air into the cell (20).

6. The cell according to claim 1 or 2 or 3 or 4 or 5, **characterised in that** the lid (50) comprises an enzymatic deodorising device (52), suitable for controlling the smell of the waste and of the vapours.

7. The cell according to claim 1 or 2 or 3 or 4 or 5, **characterised in that** it comprises a device (53) for automatically opening the lid (50).

8. The cell according to claim 1, **characterised in that** it comprises an additional set of seats (60a', 60b', 60c'), suitable for containing sensors (61a', 61b', 61c') appropriate for performing confirmation measurements of the temperatures inside the cell (20).

9. The cell according to claim 1 or 8, **characterised in that** the seats (60a, 60b, 60c; 60a', 60b', 60c') of the sensors (61a, 61b, 61c; 61a', 61b', 61c') comprise an insulating cover (62), which wraps around a sensor (61; 61'), so as to avoid contact between the sensor (61; 61') and the walls (20a) of the cell (20), and a metal cap (63) leading towards the inside of the cell (20), suitable for being in contact both with the waste and with the sensor (61; 61').

10. The cell according to claim 2, **characterised in that** the seats (70a, 70b, 70c) of the biological indicators (71a, 71b, 71c) comprise an insulating cover (72), which wraps around a biological indicator (71), so as to avoid contact between the biological indicators (71a, 71b, 71c) and the walls (20a) of the cell (20), and a metal cap (73) leading towards the inside of the cell (20), suitable for being in contact both with the waste and with the biological indicator (71).

11. The cell according to claim 5, **characterised in that** the air cooling device (51) comprises a duct (51a) for inserting air from outside into the cell (20) and a nebuliser (51b), suitable for inserting air into the cell (20).

12. The cell according to claim 6, **characterised in that** the enzymatic deodorising device (52) comprises a liquid deodorant tank (52a) outside the cell (20) and a duct (52b) for inserting the deodorant into the cell (20) leading towards a nebuliser (5 1b), in such a way as to insert the deodorant into the cell (20).
